# EUROPEAN PATENT APPLICATION

(11) **EP 1 192 985 A1**
(43) Date of publication of application: **03.04.2002**
(21) Application number: 01308124.5
(22) Date of filing: 25.09.2001
(51) Int. Cl.: B01J 23/00, B01J 23/28, B01J 27/057, B01J 37/08, C07C 253/24, C07C 51/215

(54) **Multi-metal oxide catalyst prepared using calcination**

(30) Priority: 28.09.2000 US 236029 P
(71) Applicant: ROHM AND HAAS COMPANY, Philadelphia, Pennsylvania 19106-2399 (US)
(72) Inventor: Bogan, Leonard Edward, Jr., Hatfield, Pennsylvania 19440 (US); Heffner, Michele Doreen, Chalfont, Pennsylvania 18914 (US); Jacobs, Bradley Anson, Crystal Lake, IL 60012 (US); Link, Richard David, III, Levittown, Pennsylvania 19054 (US); Vickery, Elsie Mae, Jenkintown, Pennsylvania 19046 (US)
(74) Representative: Kent, Venetia Katherine

(57) **Abstract**

A process for preparing a catalyst is disclosed. The catalyst is useful for the gas phase oxidation of alkanes to unsaturated aldehydes or carboxylic acids or for the ammoxidation of alkanes to unsaturated nitriles. The catalyst is prepared by calcination of the catalyst precursor at a temperature of at least 650°C in a non-oxidizing atmosphere. The calcination step can be carried out in at least two stages, the first stage being calcination in an oxidizing atmosphere and the second stage in a non-oxidizing atmosphere.

## Description

This invention relates to processes for preparing catalysts. In particular, the invention relates to processes for preparing catalysts which are efficient in converting alkanes to unsaturated aldehydes and carboxylic acids, or in the presence of ammonia to unsaturated nitriles; catalysts prepared by such processes and processes for preparing unsaturated aldehydes and carboxylic acids, or unsaturated nitriles, using such catalysts.

Unsaturated aldehydes and carboxylic acids are important commercial chemicals. Of particular importance is (meth)acrylic acid. The highly reactive double bond and acid function of (meth)acrylic acid makes it especially suitable as a monomer which may be polymerized alone or with other monomers to produce commercially important polymers. These unsaturated acids are also useful as a starting material for esterification to produce commercially important (meth)acrylate esters. Materials derived from (meth)acrylic acid or esters of (meth)acrylic acids are useful as plastic sheets and parts, paints and other coatings, adhesives, caulks, sealants, and detergents as well as other applications.

The production of unsaturated carboxylic acids by oxidation of an olefin is well known in the art. Acrylic acid, for instance, may be commercially manufactured by the gas phase oxidation of propylene. It is also known that unsaturated carboxylic acids may also be prepared by oxidation of alkanes. For instance, acrylic acid may be prepared by the oxidation of propane. Such a process is especially desirable because alkanes generally have a lower cost than olefins. A suitable process for the oxidation of alkanes to unsaturated aldehydes or carboxylic acids which is commercially viable has yet to be achieved.

One impediment for the production of a commercially viable process for the catalytic oxidation of an alkane to an unsaturated carboxylic acid is the identification of a catalyst having adequate conversion and suitable selectivity, thereby providing sufficient yield of the unsaturated carboxylic acid end-product.

U. S. Patent No. 5,380,933 discloses a method for preparing a catalyst useful in the gas phase oxidation of an alkane to an unsaturated carboxylic acid. In the disclosed method, a catalyst was prepared by combining ammonium metavanadate, telluric acid and ammonium paramolybdate to obtain a uniform aqueous solution. To this solution was added ammonium niobium oxalate to obtain a slurry. The water was removed from the slurry to obtain a solid catalyst precursor. The solid catalyst precursor was molded into a tablet, sieved to a desired particle size and then calcined at 600°C under a nitrogen stream to obtain the desired catalyst.

The resulting catalyst was asserted to be effective to convert propane to acrylic acid. However, as shown in European Published Patent Application No. 0 962 253 A2 (hereinafter EP 0 962 253 A2), it was not possible to reproduce the asserted results using the preparation method of the '933 patent. While not wishing to be bound by theory, it was believed that the poor performance of the prior art method of '933 resulted from compositional or phase segregation of the component elements of the catalyst, e.g., in the slurry between solid and liquid phases and during calcining between the gas and various solid phases. The aforementioned EP 0 962 253 A2 disclosed a process for preparing a catalyst for catalyzing an alkane into an unsaturated aldehyde or carboxylic acid wherein phase segregation was minimized and improvements in selectivity, conversion, and yield were achieved.

Similarly, nitriles such as acrylonitrile and methacrylonitrile, have been industrially produced as important intermediates for the preparation of fibers, synthetic resins, synthetic rubbers, and the like. The most popular method for producing such nitriles is to subject an olefin such as propene or isobutene to a catalytic reaction with ammonia and oxygen in the presence of a catalyst in a gaseous phase at a high temperature. However, in view of the price difference between propane and propene or between isobutane and isobutene, attention has been drawn to the development of a method for producing acrylonitrile or methacrylonitrile by an ammoxidation reaction wherein a lower alkane, such as propane or isobutane, is used as a starting material, and it is catalytically reacted with ammonia and oxygen in a gaseous phase in the presence of a catalyst.

Japanese Laid-Open Patent Application No. 6- 228073 discloses a method of nitrile preparation comprising reacting an alkane in a gas phase contact reaction with ammonia in the presence of a mixed metal oxide catalyst of the formula

WₐV_{b}Te_{c}XₓOₙ

wherein X represents one or more elements selected from niobium, tantalum, titanium, aluminum, zirconium, chromium, manganese, iron, ruthenium, cobaly, rhodium, nickel, palladium, platinum, antimony, bismuth, indium and cerium and, when a = 1, b = 0.01 to 1.0, c = 0.01 to 1.0, x = 0.01 to 1.0 and n is determined by the oxide form of the elements. In the working examples, the catalyst precursor was baked at 600°C under a nitrogen flow.

U.S. Patent No. 5,907,052 discloses a process for producing acrylonitrile or methacrylonitrile from propane or isobutane, respectively, by ammoxidation, which comprises reacting propane or isobutane with ammonia and molecular oxygen in the gaseous phase in a fluidized bed reactor containing a catalyst comprising a compound oxide and a silica carrier having supported thereon the compound oxide, wherein the compound oxide contains Mo, Te, V and Nb, and wherein the reaction is performed with an addition of a catalyst activator comprising at least one Te compound and, optionally, at least one Mo compound into the reactor. In the working examples, the catalyst precursor is calcined at 600°C under a nitrogen flow.

U.S. Patent No. 6,036,880 discloses a Nb-containing aqueous solution for use in producing a Nb-containing oxide catalyst, wherein the oxide catalyst comprises an oxide of a plurality of active component elements including Nb and is for use in a catalytic oxidation or ammoxidation of propane or isobutane in the gaseous phase, and wherein the oxide catalyst is prepared by a process comprising mixing the Nb-containing aqueous solution with (an) aqueous mixture(s) containing compounds of active component elements of the oxide catalyst other than Nb, to thereby provide an aqueous compound mixture, and drying the aqueous compound mixture to form a catalyst precursor, followed by calcination of the precursor. In the working examples, the catalyst precursor is calcined at 600°C under a nitrogen flow.

It has now been determined that if the calcination of the catalyst precursor of the mixed metal oxide containing catalyst as hereinafter defined is carried out at a temperature of at least 650°C in a non-oxidizing atmosphere, or if the calcination of the catalyst precursor of the mixed metal oxide containing catalyst as hereinafter defined is carried out in at least two stages, the first stage being calcination in an oxidizing atmosphere and the second stage being calcination in a non-oxidizing atmosphere, the mixed metal oxide containing catalyst exhibits improved selectivity toward the desired oxidation product in the oxidation of an alkane to produce an unsaturated carboxylic acid and in the ammoxidation of an alkane to produce an unsaturated nitrile.

Accordingly, in one aspect of the present invention, there is provided an improved process for preparing a catalyst including a compound having the formula

AₐV_{b}N_{c}X_{d}Oₑ

wherein, when a = 1, b = 0.1 to 1, c = 0.1 to 1, d = 0.01 to 1 and e is dependent on the oxidation state of the other elements, and
wherein A is at least one element selected from the group consisting of Mo and W, N is at least one element selected from the group consisting of Te, Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb, said process comprising:
admixing metal compounds, at least one of which is an oxygen containing compound, and at least one solvent to form a solution;
removing the solvent from the solution to obtain a catalyst precursor; and
calcining the catalyst precursor to form said catalyst;
   wherein the improvement comprises:
   (A) admixing said metal compounds, at least one of which is an oxygen containing compound, and said at least one solvent to form a solution,
   (B) removing said at least one solvent from said solution to obtain a catalyst precursor, and
   (C) calcining said catalyst precursor at a temperature of at least 650°C in a non-oxidizing atmosphere to form a catalyst including a compound having the formula

      AₐV_{b}N_{c}X_{d}Oₑ
   wherein, when a = 1, b = 0.1 to 1, c = 0.1 to 1, d = 0.01 to 1 and e is dependent on the oxidation state of the other elements, and
   wherein A is at least one element selected from the group consisting of Mo
   and W, N is at least one element selected from the group consisting of Te,
   Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb.

In a second aspect of the present invention, there is provided an improved process for preparing a catalyst including a compound having the formula

AₐV_{b}N_{c}X_{d}Oₑ

wherein, when a = 1, b = 0.1 to 1, c = 0.1 to 1, d = 0.01 to 1 and e is dependent on the oxidation state of the other elements, and
wherein A is at least one element selected from the group consisting of Mo and W, N is at least one element selected from the group consisting of Te, Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb, said process comprising:
admixing metal compounds, at least one of which is an oxygen containing compound, and at least one solvent to form a solution;
removing the solvent from the solution to obtain a catalyst precursor; and
calcining the catalyst precursor to form said catalyst;
   wherein the improvement comprises:
   (A) admixing said metal compounds, at least one of which is an oxygen containing compound, and said at least one solvent to form a solution,
   (B) removing said at least one solvent from said solution to obtain a catalyst precursor, and
   (C) heating said catalyst precursor to a first temperature in an oxidizing atmosphere, then heating the so-treated catalyst precursor from said first temperature to a second temperature in a non-oxidizing atmosphere to form a catalyst including a compound having the formula

      AₐV_{b}N_{c}X_{d}Oₑ
   wherein, when a = 1, b = 0.1 to 1, c = 0.1 to 1, d = 0.01 to 1 and e is dependent on the oxidation state of the other elements, and
   wherein A is at least one element selected from the group consisting of Mo
   and W, N is at least one element selected from the group consisting of Te,
   Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb.

In a third aspect, the present invention provides a catalyst including a compound of the formula:

AₐV_{b}N_{c}X_{d}Oₑ

wherein, when a = 1, b = 0.1 to 1, c = 0.1 to 1, d = 0.01 to 1 and e is dependent on the oxidation state of the other elements, and
wherein A is at least one element selected from the group consisting of Mo and W, N is at least one element selected from the group consisting of Te, Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb,produced by the process comprising
(A) admixing metal compounds, at least one of which is an oxygen containing compound, and at least one solvent to form a solution,
(B) removing said at least one solvent from said solution to obtain a catalyst precursor, and
(C) calcining said catalyst precursor at a temperature of at least 650°C in a non-oxidizing atmosphere.

In a fourth aspect, the invention provides a catalyst including a compound of the formula

AₐV_{b}N_{c}X_{d}Oₑ

wherein, when a = 1, b = 0.1 to 1, c = 0.1 to 1, d = 0.1 to 1 and e is dependent on the oxidation state of the other elements, and
wherein A is at least one element selected from the group consisting of Mo and W, N is at least one element selected from the group consisting of Te, Se and Sb, and X is at least one element selected from the group consisting of Nb, Ta, Ti, Al, Zr, Cr, Mn, Fe, Ru, Co, Rh, Ni, Pd, Pt, Bi,, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb, produced by the process comprising:
(A) admixing metal compounds, at least one of which is an oxygen containing compound, and at least one solvent to form a solution,
(B) removing said at least one solvent from said solution to obtain a catalyst precursor, and
(C) heating said catalyst precursor to a first temperature in an oxidizing atmosphere, then heating the so-treated catalyst precursor from said first temperature to a second temperature in a non-oxidizing atmosphere.

In additional aspects of the present invention, there are provided processes for preparing unsaturated aldehydes or carboxylic acids, including subjecting an alkane to catalytic oxidation in the presence of a catalyst prepared according to the present invention; or for preparing unsaturated nitriles, including subjecting an alkane to catalytic oxidation in the presence of ammonia and a catalyst prepared according to the present invention.

Figure 1 is a plot of propane conversion versus product selectivity (to acrylic acid) for various catalysts used in the vapor phase oxidation of propane to acrylic acid wherein the catalysts were calcined solely under a non-oxidizing atmosphere.

Figure 2 is a plot of propane conversion versus product selectivity (to acrylic acid) for various catalysts used in the vapor phase oxidation of propane to acrylic acid wherein the catalysts were calcined first under an oxidizing atmosphere and then under a non-oxidizing atmosphere.

As used herein, the expression "(meth)acrylic acid" is intended to include both methacrylic acid and acrylic acid within its scope. In a like manner, the expression "(meth)acrylates" is intended to include both methacrylates and acrylates within its scope. Similarly, the expression "(meth)acrylonitrile" is intended to include both methacrylonitrile and acrylonitrile within its scope.

As used herein the terminology "(C₃-C₈) alkane" means a straight chain or branched chain alkane having from 3 to 8 carbon atoms per alkane molecule.

As used herein the term "mixture" is meant to include within its scope all forms of mixtures including, but not limited to, simple mixtures as well as blends, alloys, etc.

For purposes of this application "% conversion" is equal to (moles of consumed alkane/moles of supplied alkane) X 100; "% selectivity" is equal to (moles of formed desired unsaturated product(s)/moles of consumed alkane) X 100; and "%yield" is equal to (moles of formed desired unsaturated product(s)/moles of supplied alkane) X 100.

For purposes of this application by "solution" is meant that greater than 95 percent of metal solid added to a solvent is dissolved. It is to be understood that the greater the amount of metal solid not initially in solution, the poorer the performance of the catalyst derived therefrom will be.

As recited above, a process for preparing a catalyst is disclosed. In a first step of the process a solution is formed by admixing metal compounds, at least one of which contains oxygen, and at least one solvent in appropriate amounts to form the solution. Generally, the metal compounds contain elements A, V, N, X, and O. In one preferred embodiment, A is at least one element selected from the group consisting of Mo and W; N is at least one element selected from the group consisting of Te and Sb; and X is at least one element selected from the group consisting of Nb, Ta and, Zr. In a more preferred embodiment, A is Mo, N is Te and X is Nb.

Suitable solvents include water, alcohols including, but not limited to, methanol, ethanol, propanol, and diols etc, as well as other polar solvents known in the art. Generally, water is preferred. The water is any water suitable for use in chemical synthesis including, without limitation, distilled water and deionized water. The amount of water present is that amount sufficient to keep the elements substantially in solution long enough to avoid or minimize compositional and/or phase segregation during the preparation steps. Accordingly, the amount of water will vary according to the amounts and solubility of materials combined. However, as stated above, it is preferred that the amount of water be sufficient to insure an aqueous solution is formed rather than a slurry at the time of mixing.

For example, when a mixed metal oxide of the formula MoₐV_{b}Te_{c}Nb_{d}Oₑ, wherein the element A is Mo, the element N is Te and the element X is Nb, is to be prepared, an aqueous solution of niobium oxalate may be added to an aqueous solution of ammonium heptamolybdate, ammonium metavanadate and telluric acid, so that the atomic ratio of the respective metal elements would be in the presribed proportions.

Once the mixture is formed, the solvent is removed by any suitable method known in the art to form a catalyst precursor. Such methods include, without limitation, vacuum drying, freeze drying, spray drying, rotary evaporation, and air drying. Vacuum drying is generally performed at pressures ranging from 10 to 500 mm/Hg. Freeze drying typically entails freezing the solution, using for instance liquid nitrogen, and drying the frozen solution under vacuum. Spray drying is generally performed under an inert atmosphere such as nitrogen or argon, with an inlet temperature ranging from 125°C to 200°C and an outlet temperature ranging from 75°C to 150°C. Rotary evaporation is generally performed at a bath temperature of from 25°C to 90°C and a pressure of from 10 mm/Hg to 760 mm/Hg, preferably at a bath temperature of from 40°C to 90°C and a pressure from 10 mm/Hg to 350 mm/Hg, more preferably from 40°C to 60°C and a pressure of from 10 mm/Hg to 40 mm/Hg. Air drying may be occur at temperatures ranging from 25°C to 90°C. Rotary evaporation or air drying are generally preferred.

Once obtained, the catalyst precursor is calcined at a temperature of at least 650°C in a non-oxidizing atmosphere. The non-oxidizing atmosphere may be any material which will not cause oxidation of the catalyst precursor when contacted with the catalyst precursor under the defined calcination condition. Typically, the non-oxidizing atmosphere will comprise an inert gas, i.e., a gas that does not react or interact with the catalyst precursor. Suitable examples include, without limitation, nitrogen, argon, xenon, helium or mixtures thereof. Preferably, the inert gas is argon or nitrogen, more preferably argon. Optionally, a reducing gas, such as, for example, ammonia or hydrogen, may be included in the non-oxidizing atmosphere. The non-oxidizing atmosphere preferably may flow over the surface of the catalyst precursor. The flow rate of the non-oxidizing atmosphere can vary over a wide range, for example, space velocities of from 1 to 1,000 hr⁻¹ or more can be utilized.

The calcination in a non-oxidizing atmosphere is performed at a temperature of at least 650°C, e.g., at a temperature of from 650°C to 850°C, preferably from 650°C to 750°C, more preferably from 650°C to 700°C. The calcination is performed for an amount of time suitable to form the aforementioned catalyst, e.g., for from 0.5 to 20 hours, preferably for from 1 to 10 hours and more preferably for from 1 to 5 hours.

Alternatively, the catalyst precursor may be calcined in two or more stages. Each calcination stage may be defined by calcination temperature and/or calcination atmosphere. For example, in a first calcination stage, the catalyst precursor could be calcined at a first temperature in a non-oxidizing atmosphere and then, in a second calcination stage, the so-treated material could be calcined at a higher second temperature in a non-oxidizing atmosphere. Alternatively, in a first calcination stage the catalyst precursor could be calcined in an oxidizing atmosphere and then, in a second calcination stage, the so-treated material could be calcined in a non-oxidizing atmosphere. The sole requirement, in any case, would be that, once the material has been calcined in a stage having a non-oxidizing atmosphere, it not be again subjected only to a calcination stage in an oxidizing atmosphere.

In a preferred mode of operation, the catalyst precursor may be calcined under a flowing atmosphere in two stages. In the first stage, the catalyst precursor is calcined in a flowing oxidizing atmosphere. The oxidizing atmosphere may be any material which will oxidize the catalyst precursor when contacted with the catalyst precursor under the defined conditions. Typically, the oxidizing atmosphere will comprise an oxidizing gas such as oxygen, air, nitrogen monoxide or an oxygen enriched gas. The first stage calcination may be carried out at a temperature of from 200°C to 400°C, preferably from 275°C to 325°C, for from 15 minutes to 8 hours, preferably for from 1 to 3 hours. In the second stage, the material from the first stage is calcined in a flowing non-oxidizing atmosphere at a temperature of at least 500°C, preferably from 600°C to 750°C, more preferably from 650°C to 700°C, for from 15 minutes to 8 hours, preferably for from 1 to 3 hours.

In a particularly preferred mode of operation, the catalyst precursor in the first stage of calcination is placed in the desired flowing oxidizing atmosphere at room temperature and then raised to the first stage calcination temperature, at a rate of from 1°C/min to 20°C/min, preferably 2°C/min to 10°C/min. It is then held at the first stage calcination temperature, in the desired flowing oxidizing atmosphere, for the desired first stage calcination time. After the desired first stage calcination time has passed, the atmosphere is replaced with the desired flowing non-oxidizing atmosphere, preferably while maintaining the temperature at the first stage calcination temperature; the temperature is then raised to the desired second stage calcination temperature at a rate of from 1°C/min to 20°C/min, preferably 2°C/min to 10°C/min. It is then held at the second stage calcination temperature, in the desired flowing non-oxidizing atmosphere, for the desired second stage calcination time.

With calcination a catalyst is formed including a compound having the formula

AₐV_{b}N_{c}X_{d}Oₑ

wherein A, N and X are as previously defined, and wherein when a = 1, b = 0.01 to 1, c = 0.01 to 1 and d = 0.01 to 1, preferably b = 0.1 to 0.5, c = 0.05 to 0.5 and d = 0.01 to 0.5, more preferably b = 0.15 to 0.45, c= 0.05 to 0.45 and d = 0.01 to 0.1; and e, the molar ratio of the amount of oxygen (O) present, is dependent on the oxidation state of the other elements in the compound. Typically, e is from 3 to 4.7, based on the other elements present in the compound.

The starting materials for the above mixed metal oxide are not limited to those described above. A wide range of materials including, for example, oxides, nitrates, halides or oxyhalides, alkoxides, acetylacetonates and organometallic compounds may be used. For example, ammonium heptamolybdate may be utilized for the source of molybdenum in the catalyst. However, compounds such as MoO₃, MoO₂, MoCl₅, MoOCl₄, Mo(OC₂H₅)₅, molybdenum acetylacetonate, phosphomolybdic acid and silicomolybdic acid may also be utilized instead of ammonium heptamolybdate. Similarly, ammonium metavanadate may be utilized for the source of vanadium in the catalyst. However, compounds such as V₂O₅, V₂O₃, VOCl₃, VCl₄, VO(OC₂H₅)₃, vanadium acetylacetonate and vanadyl acetylacetonate may also be utilized instead of ammonium metavanadate. The tellurium source may include telluric acid, TeCl₄, Te(OC₂H₅)₅, Te(OCH(CH₃)₂)₄ and TeO₂. The niobium source may include ammonium niobium oxalate, Nb₂O₅, NbCl₅, niobic acid or Nb(OC₂H₅)₅ as well as the more conventional niobium oxalate.

The mixed metal oxide, thus obtained, exhibits excellent catalytic activities by itself. However, the mixed metal oxide can be converted to a catalyst having higher activities by grinding.

There is no particular restriction as to the grinding method, and conventional methods may be employed. As a dry grinding method, a method of using a gas stream grinder may, for example, be mentioned wherein coarse particles are permitted to collide with one another in a high speed gas stream for grinding. The grinding may be conducted not only mechanically but also by using a mortar or the like in the case of a small scale operation.

As a wet grinding method wherein grinding is conducted in a wet state by adding water or an organic solvent to the above mixed metal oxide, a conventional method of using a rotary cylinder-type medium mill or a medium-stirring type mill, may be mentioned. The rotary cylinder-type medium mill is a wet mill of the type wherein a container for the object to be ground is rotated, and it includes, for example, a ball mill and a rod mill. The medium-stirring type mill is a wet mill of the type where the object to be ground, contained in a container, is stirred by a stirring apparatus, and it includes, for example, a rotary screw type mill and a rotary disc type mill.

The conditions for grinding may suitably be set to meet the nature of the above-mentioned mixed metal oxide, the viscosity, the concentration, etc. of the solvent used in the case of wet grinding, or the optimum conditions of the grinding apparatus. However, it is preferred that grinding is conducted until the average particle size of the ground catalyst precursor would usually be at most 20µm, more preferably at most 5µm. Improvement in the catalytic performance may occur due to such grinding.

Further, in some cases, it is possible to further improve the catalytic activities by further adding a solvent to the ground catalyst precursor to form a solution or slurry, followed by drying again. There is no particular restriction as to the concentration of the solution or slurry, and it is usual to adjust the solution or slurry so that the total amount of the starting material compounds for the ground catalyst precursor is from 10 to 60 wt %. Then, this solution or slurry is dried by a method such as spray drying, freeze drying, evaporation to dryness or vacuum drying, preferably by the spray drying method. Further, similar drying may be conducted also in the case where wet grinding is conducted.

The oxide obtained by the above-mentioned method may be used as a final catalyst, but it may firther be subjected to heat treatment usually at a temperature of from 200°C to 700° for from 0.1 to 10 hours.

The third and fourth aspects of the present invention are catalysts for manufacturing an unsaturated aldehyde or a carboxylic acid from an alkane, or for manufacturing an unsaturated nitrile from an alkane, prepared by the processes of the present invention. The catalysts are prepared as described above. The catalysts may be used as solid catalysts alone or may be formed into catalysts together with a suitable carrier such as silica, alumina, titania, aluminosilicate, diatomaceous earth or zirconia. The shape of the catalyst can be any suitable shape and will depend upon the particular application of the catalyst. In a like manner, the particle size of the catalyst may be any suitable particle size depending on the particular use of the catalyst.

Alternatively, the metal components of the presently contemplated catalyst may be supported on materials such as alumina, silica, silica-alumina, zirconia, titania, etc. by conventional incipient wetness techniques. In one typical method, solutions containing the metals are contacted with the dry support such that the support is wetted; then, the resultant wetted material is dried, for example, at a temperature from room temperature to 200°C followed by calcination as described above. In another method, metal solutions are contacted with the support, typically in volume ratios of greater than 3 : 1 (metal solution : support), and the solution agitated such that the metal ions are ion-exchanged onto the support. The metal-containing support is then dried and calcined as detailed above.

In a further aspect, the present invention provides a process for producing an unsaturated carboxylic acid, which comprises subjecting an alkane, or a mixture of an alkane and an alkene, to a vapor phase catalytic oxidation reaction in the presence of a catalyst containing the above promoted mixed metal oxide, to produce an unsaturated carboxylic acid.

In the production of such an unsaturated carboxylic acid, it is preferred to employ a starting material gas which contains steam. In such a case, as a starting material gas to be supplied to the reaction system, a gas mixture comprising a steam-containing alkane, or a steam-containing mixture of alkane and alkene, and an oxygen-containing gas, is usually used. However, the steam-containing alkane, or the steam-containing mixture of alkane and alkene, and the oxygen-containing gas may be alternately supplied to the reaction system. The steam to be employed may be present in the form of steam gas in the reaction system, and the manner of its introduction is not particularly limited.

Further, as a diluting gas, an inert gas such as nitrogen, argon or helium may be supplied. The molar ratio (alkane or mixture of alkane and alkene) : (oxygen) : (diluting gas) : (H₂O) in the starting material gas is preferably (1) : (0.1 to 10): (0 to 20): (0.2 to 70), more preferably (1): (1 to 5.0): (0 to 10): (5 to 40).

When steam is supplied together with the alkane, or the mixture of alkane and alkene, as starting material gas, the selectivity for an unsaturated carboxylic acid is distinctly improved, and the unsaturated carboxylic acid can be obtained from the alkane, or mixture of alkane and alkene, in good yield simply by contacting in one stage. However, the conventional technique utilizes a diluting gas such as nitrogen, argon or helium for the purpose of diluting the starting material. As such a diluting gas, to adjust the space velocity, the oxygen partial pressure and the steam partial pressure, an inert gas such as nitrogen, argon or helium may be used together with the steam.

As the starting material alkane it is preferred to employ a C₃₋₈alkane, particularly propane, isobutane or n-butane; more preferably, propane or isobutane; most preferably, propane. According to the present invention, from such an alkane, an unsaturated carboxylic acid such as an α,β-unsaturated carboxylic acid can be obtained in good yield. For example, when propane or isobutane is used as the starting material alkane, acrylic acid or methacrylic acid will be obtained, respectively, in good yield.

In the present invention, as the starting material mixture of alkane and alkene, it is preferred to employ a mixture of C₃₋₈alkane and C₃₋₈alkene, particularly propane and propene, isobutane and isobutene or n-butane and n-butene. As the starting material mixture of alkane and alkene, propane and propene or isobutane and isobutene are more preferred. Most preferred is a mixture of propane and propene. According to the present invention, from such a mixture of an alkane and an alkene, an unsaturated carboxylic acid such as an α,β-unsaturated carboxylic acid can be obtained in good yield. For example, when propane and propene or isobutane and isobutene are used as the starting material mixture of alkane and alkene, acrylic acid or methacrylic acid will be obtained, respectively, in good yield. Preferably, in the mixture of alkane and alkene, the alkene is present in an amount of at least 0.5% by weight, more preferably at least 1.0% by weight to 95% by weight; most preferably, 3% by weight to 90% by weight.

As an alternative, an alkanol, such as isobutanol, which will dehydrate under the reaction conditions to form its corresponding alkene, i.e. isobutene, may also be used as a feed to the present process or in conjunction with the previously mentioned feed streams.

The purity of the starting material alkane is not particularly limited, and an alkane containing a lower alkane such as methane or ethane, air or carbon dioxide, as impurities, may be used without any particular problem. Further, the starting material alkane may be a mixture of various alkanes. Similarly, the purity of the starting material mixture of alkane and alkene is not particularly limited, and a mixture of alkane and alkene containing a lower alkene such as ethene, a lower alkane such as methane or ethane, air or carbon dioxide, as impurities, may be used without any particular problem. Further, the starting material mixture of alkane and alkene may be a mixture of various alkanes and alkenes.

There is no limitation on the source of the alkene. It may be purchased, per se, or in admixture with an alkane and/or other impurities. Alternatively, it can be obtained as a by-product of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

The detailed mechanism of the oxidation reaction of the present invention is not clearly understood, but the oxidation reaction is carried out by oxygen atoms present in the above promoted mixed metal oxide or by molecular oxygen present in the feed gas. To incorporate molecular oxygen into the feed gas, such molecular oxygen may be pure oxygen gas. However, it is usually more economical to use an oxygen-containing gas such as air, since purity is not particularly required.

It is also possible to use only an alkane, or a mixture of alkane and alkene, substantially in the absence of molecular oxygen for the vapor phase catalytic reaction. In such a case, it is preferred to adopt a method wherein a part of the catalyst is appropriately withdrawn from the reaction zone from time to time, then sent to an oxidation regenerator, regenerated and then returned to the reaction zone for reuse. As the regeneration method of the catalyst, a method may, for example, be mentioned which comprises contacting an oxidative gas such as oxygen, air or nitrogen monoxide with the catalyst in the regenerator usually at a temperature of from 300° to 600°C.

This aspect of the present invention will be described in further detail with respect to a case where propane is used as the starting material alkane and air is used as the oxygen source. The reaction system may be a fixed bed system or a fluidized bed system. However, since the reaction is an exothermic reaction, a fluidized bed system may preferably be employed whereby it is easy to control the reaction temperature. The proportion of air to be supplied to the reaction system is important for the selectivity for the resulting acrylic acid, and it is usually at most 25 moles, preferably from 0.2 to 18 moles per mole of propane, whereby high selectivity for acrylic acid can be obtained. This reaction can be conducted usually under atmospheric pressure, but may be conducted under a slightly elevated pressure or slightly reduced pressure. With respect to other alkanes such as isobutane, or to mixtures of alkanes and alkenes such as propane and propene, the composition of the feed gas may be selected in accordance with the conditions for propane.

Typical reaction conditions for the oxidation of propane or isobutane to acrylic acid or methacrylic acid may be utilized in the practice of the present invention. The process may be practiced in a single pass mode (only fresh feed is fed to the reactor) or in a recycle mode (at least a portion of the reactor effluent is returned to the reactor). General conditions for the process of the present invention are as follows: the reaction temperature can vary from 200°C to 700°C, but is usually in the range of from 200°C to 550°C, more preferably 250°C to 480°C, most preferably 300°C to 400°C; the gas space velocity, SV, in the vapor phase reaction is usually within a range of from 100 to 10,000 hr⁻¹, preferably 300 to 6,000 hr⁻¹, more preferably 300 to 2,000 hr⁻¹; the average contact time with the catalyst can be from 0.01 to 10 seconds or more, but is usually in the range of from 0.1 to 10 seconds, preferably from 2 to 6 seconds; the pressure in the reaction zone usually ranges from 0 to 75 psig, but is preferably no more than 50 psig. In a single pass mode process, it is preferred that the oxygen be supplied from an oxygen-containing gas such as air. The single pass mode process may also be practiced with oxygen addition. In the practice of the recycle mode process, oxygen gas by itself is the preferred source so as to avoid the build up of inert gases in the reaction zone.

Of course, in the oxidation reaction of the present invention, it is important that the hydrocarbon and oxygen concentrations in the feed gases be maintained at the appropriate levels to minimize or avoid entering a flammable regime within the reaction zone or especially at the outlet of the reactor zone. Generally, it is preferred that the outlet oxygen levels be low to both minimize after-burning and, particularly, in the recycle mode of operation, to minimize the amount of oxygen in the recycled gaseous effluent stream. In addition, operation of the reaction at a low temperature (below 450°C) is extremely attractive because after-burning becomes less of a problem which enables the attainment of higher selectivity to the desired products. The catalyst of the present invention operates more efficiently at the lower temperature range set forth above, significantly reducing the formation of acetic acid and carbon oxides, and increasing selectivity to acrylic acid. As a diluting gas to adjust the space velocity and the oxygen partial pressure, an inert gas such as nitrogen, argon or helium may be employed.

When the oxidation reaction of propane, and especially the oxidation reaction of propane and propene, is conducted by the method of the present invention, carbon monoxide, carbon dioxide, acetic acid, etc. may be produced as by-products, in addition to acrylic acid. Further, in the method of the present invention, an unsaturated aldehyde may sometimes be formed depending upon the reaction conditions. For example, when propane is present in the starting material mixture, acrolein may be formed; and when isobutane is present in the starting material mixture, methacrolein may be formed. In such a case, such an unsaturated aldehyde can be converted to the desired unsaturated carboxylic acid by subjecting it again to the vapor phase catalytic oxidation with the promoted mixed metal oxide-containing catalyst of the present invention or by subjecting it to a vapor phase catalytic oxidation reaction with a conventional oxidation reaction catalyst for an unsaturated aldehyde.

In a still further aspect, the method of the present invention comprises subjecting an alkane, or a mixture of an alkane and an alkene, to a vapor phase catalytic oxidation reaction with ammonia in the presence of a catalyst containing the above mixed metal oxide, to produce an unsaturated nitrile.

In the production of such an unsaturated nitrile, as the starting material alkane, it is preferred to employ a C₃₋₈alkane such as propane, butane, isobutane, pentane, hexane and heptane. However, in view of the industrial application of nitriles to be produced, it is preferred to employ a lower alkane having 3 or 4 carbon atoms, particularly propane and isobutane.

Similarly, as the starting material mixture of alkane and alkene, it is preferred to employ a mixture of C₃₋₈alkane and C₃₋₈alkene such as propane and propene, butane and butene, isobutane and isobutene, pentane and pentene, hexane and hexene, and heptane and heptene. However, in view of the industrial application of nitriles to be produced, it is more preferred to employ a mixture of a lower alkane having 3 or 4 carbon atoms and a lower alkene having 3 or 4 carbon atoms, particularly propane and propene or isobutane and isobutene. Preferably, in the mixture of alkane and alkene, the alkene is present in an amount of at least 0.5% by weight, more preferably at least 1.0% by weight to 95% by weight, most preferably 3% by weight to 90% by weight.

The purity of the starting material alkane is not particularly limited, and an alkane containing a lower alkane such as methane or ethane, air or carbon dioxide, as impurities, may be used without any particular problem. Further, the starting material alkane may be a mixture of various alkanes. Similarly, the purity of the starting material mixture of alkane and alkene is not particularly limited, and a mixture of alkane and alkene containing a lower alkene such as ethene, a lower alkane such as methane or ethane, air or carbon dioxide, as impurities, may be used without any particular problem. Further, the starting material mixture of alkane and alkene may be a mixture of various alkanes and alkenes.

There is no limitation on the source of the alkene. It may be purchased, per se, or in admixture with an alkane and/or other impurities. Alternatively, it can be obtained as a by-product of alkane oxidation. Similarly, there is no limitation on the source of the alkane. It may be purchased, per se, or in admixture with an alkene and/or other impurities. Moreover, the alkane, regardless of source, and the alkene, regardless of source, may be blended as desired.

The detailed mechanism of the ammoxidation reaction of this aspect of the present invention is not clearly understood. However, the oxidation reaction is conducted by the oxygen atoms present in the above promoted mixed metal oxide or by the molecular oxygen in the feed gas. When molecular oxygen is incorporated in the feed gas, the oxygen may be pure oxygen gas. However, since high purity is not required, it is usually economical to use an oxygen-containing gas such as air.

As the feed gas, it is possible to use a gas mixture comprising an alkane, or a mixture of an alkane and an alkene, ammonia and an oxygen-containing gas, However, a gas mixture comprising an alkane or a mixture of an alkane and an alkene and ammonia, and an oxygen-containing gas may be supplied alternately.

When the gas phase catalytic reaction is conducted using an alkane, or a mixture of an alkane and an alkene, and ammonia substantially free from molecular oxygen, as the feed gas, it is advisable to employ a method wherein a part of the catalyst is periodically withdrawn and sent to an oxidation regenerator for regeneration, and the regenerated catalyst is returned to the reaction zone. As a method for regenerating the catalyst, a method may be mentioned wherein an oxidizing gas such as oxygen, air or nitrogen monoxide is permitted to flow through the catalyst in the regenerator usually at a temperature of from 300°C to 600°C.

This still further aspect of the present invention will be described in further detail with respect to a case where propane is used as the starting material alkane and air is used as the oxygen source. The proportion of air to be supplied for the reaction is important with respect to the selectivity for the resulting acrylonitrile. Namely, high selectivity for acrylonitrile is obtained when air is supplied within a range of at most 25 moles, particularly 1 to 15 moles, per mole of the propane. The proportion of ammonia to be supplied for the reaction is preferably within a range of from 0.2 to 5 moles, particularly from 0.5 to 3 moles, per mole of propane. This reaction may usually be conducted under atmospheric pressure, but may be conducted under a slightly increased pressure or a slightly reduced pressure. With respect to other alkanes such as isobutane, or to mixtures of alkanes and alkenes such as propane and propene, the composition of the feed gas may be selected in accordance with the conditions for propane.

The process of the third aspect of the present invention may be conducted at a temperature of, for example, from 250°C to 480°C. More preferably, the temperature is from 300°C to 400°C. The gas space velocity, SV, in the gas phase reaction is usually within the range of from 100 to 10,000 hr⁻¹, preferably from 300 to 6,000 hr⁻¹, more preferably from 300 to 2,000 hr⁻¹. As a diluent gas, for adjusting the space velocity and the oxygen partial pressure, an inert gas such as nitrogen, argon or helium can be employed. When ammoxidation of propane is conducted by the method of the present invention, in addition to acrylonitrile, carbon monoxide, carbon dioxide, acetonitrile, hydrocyanic acid and acrolein may form as by-products.

### Examples

### Example 1

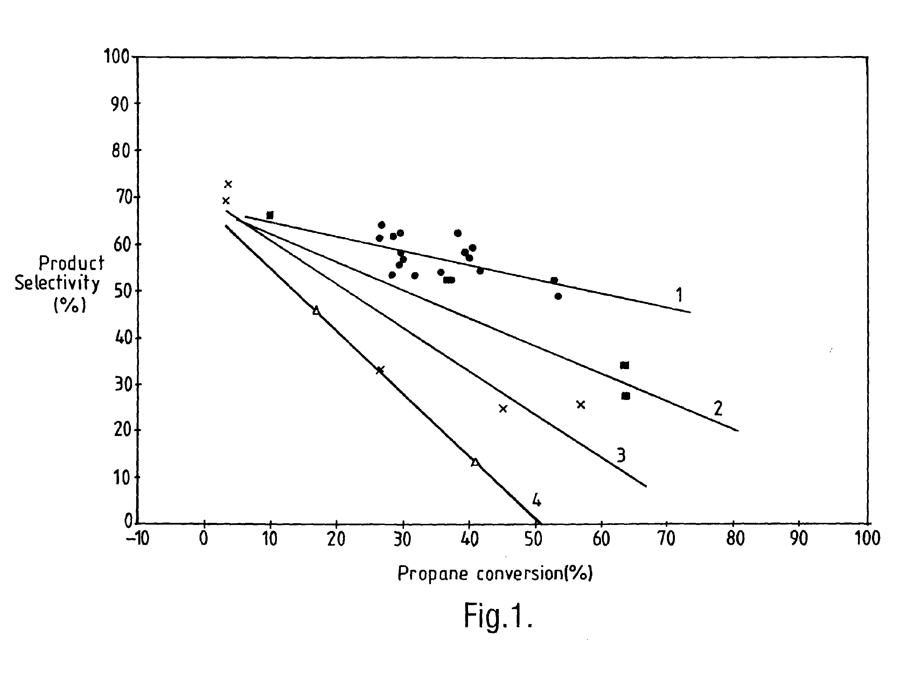

An aqueous solution containing ammonium heptamolybdate tetrahydrate, ammonium metavanadate and telluric acid was prepared by dissolving the corresponding salts in water at 70°C. The so-formed solution was cooled to 40°C and an aqueous solution of niobium oxalate was added thereto. After removing the water therefrom at 50°C, under 95 to 35 mmHg, the solid materials were further dried in a vacuum oven at 70°C overnight. The dried materials were placed in a crucible with the lid ajar. The crucible was then placed in a 1000mL quartz beaker filled with 1 inch of sand. The beaker was covered with a porcelain lid having a small (1/4") hole in the center and placed in a calcining furnace. A stainless steel tube was connected to an argon supply and place in the hole in the lid. The beaker was purged with argon (8 standard cubic feet per hour) for 30 minutes at room temperature. Then, the crucible lid was closed and heating started while maintaining the argon flow at 2 - 3 standard cubic feet per hour. The furnace was heated at 2°C/min to 200°C and then held at that temperature for 1 hour; heating was then continued at 2°C/min to 700°C and then held at that temperature for 2 hours. After cooling to room temperature, the final catalyst, which had a nominal composition of Mo₁V_{0.30}Te_{0.23}Nb_{0.08}, was pressed in a mold and then broken and sieved to 10 -20 mesh granules for reactor evaluation, i.e. the vapor phase catalytic oxidation of propane to acrylic acid. The reactor evaluation results are plotted as curve 1 (solid circles) in Fig. 1.

### Comparative Example 1

An aqueous solution containing ammonium heptamolybdate tetrahydrate, ammonium metavanadate and telluric acid was prepared by dissolving the corresponding salts in water at 70C°. The so-formed solution was cooled to 40°C and an aqueous solution of niobium oxalate was added thereto. After removing the water therefrom at 50°C, under 100 to 40 mmHg, the solid materials were further dried in a vacuum oven at 25°C overnight. The dried materials were placed in a crucible with the lid ajar. The crucible was then placed in a 1000mL quartz beaker filled with 1 inch of sand. The beaker was covered with a porcelain lid having a small (1/4") hole in the center and placed in a calcining furnace. A stainless steel tube was connected to an argon supply and placed in the hole in the lid. The beaker was purged with argon (8 standard cubic feet per hour) for 30 minutes at room temperature. Then, the crucible lid was closed and heating started while maintaining the argon flow at 2 - 3 scfh. The furnace was heated at 2°C/min to 200°C and then held at that temperature for 1 hour; heating was then continued at 2°C/min to 600°C and then held at that temperature for 16 hours. After cooling to room temperature, the final catalyst, which had a nominal composition of Mo₁V_{0.30}Te_{0.23}Nb_{0.08}, was pressed in a mold and then broken and sieved to 10 -20 mesh granules for reactor evaluation, i.e. the vapor phase catalytic oxidation of propane to acrylic acid. The reactor evaluation results are plotted as curve 2 (solid squares) in Fig. 1.

### Comparative Example 2

An aqueous solution containing ammonium heptamolybdate tetrahydrate, ammonium metavanadate and telluric acid was prepared by dissolving the corresponding salts in water at 70C°. The so-formed solution was cooled to 40°C and an aqueous solution of niobium oxalate was added thereto. After removing the water therefrom at 50°C, under 100 to 40 mmHg, the solid materials were further dried in a vacuum oven at 25°C overnight. The dried materials were placed in a crucible with the lid ajar. The crucible was then placed in a 1000mL quartz beaker filled with 1 inch of sand. The beaker was covered with a porcelain lid having a small (1/4") hole in the center and placed in a calcining furnace. A stainless steel tube was connected to an argon supply and placed in the hole in the lid. The beaker was purged with argon (8scfh) for 30 minutes at room temperature. Then, the crucible lid was closed and heating started while maintaining the argon flow at 2 - 3 scfh. The furnace was heated at 2°C/min to 200°C and then held at that temperature for 1 hour; heating was then continued at 2°C/min to 550°C and then held at that temperature for 16 hours. After cooling to room temperature, the final catalyst, which had a nominal composition of Mo₁V_{0.30}Te_{0.23}Nb_{0.08}, was pressed in a mold and then broken and sieved to 10 - 20 mesh granules for reactor evaluation, i.e. the vapor phase catalytic oxidation of propane to acrylic acid. The reactor evaluation results are plotted as curve 3 (X's) in Fig. 1.

### Comparative Example 3

An aqueous solution containing ammonium heptamolybdate tetrahydrate, ammonium metavanadate and telluric acid was prepared by dissolving the corresponding salts in water at 70C°. The so-formed solution was cooled to 40°C and an aqueous solution of niobium oxalate was added thereto. After removing the water therefrom at 50°C, under 100 to 40 mmHg, the solid materials were further dried in a vacuum oven at 70°C overnight. The dried materials were placed in a crucible with the lid ajar. The crucible was then placed in a 1000mL quartz beaker filled with 1 inch of sand. The beaker was covered with a porcelain lid having a small (1/4") hole in the center and placed in a calcining furnace. A stainless steel tube was connected to an argon supply and placed in the hole in the lid. The beaker was purged with argon (8scfh) for 30 minutes at room temperature. Then, the crucible lid was closed and heating started while maintaining the argon flow at 2 - 3 scfh. The furnace was heated at 2°C/min to 200°C and then held at that temperature for 1 hour; heating was then continued at 2°C/min to 450°C and then held at that temperature for 16 hours. After cooling to room temperature, the final catalyst, which had a nominal composition of Mo₁V_{0.30}Te_{0.23}Nb_{0.08}, was pressed in a mold and then broken and sieved to 10 - 20 mesh granules for reactor evaluation, i.e. the vapor phase catalytic oxidation of propane to acrylic acid. The reactor evaluation results are plotted as curve 4 (open triangles) in Fig. 1.

### Example 2

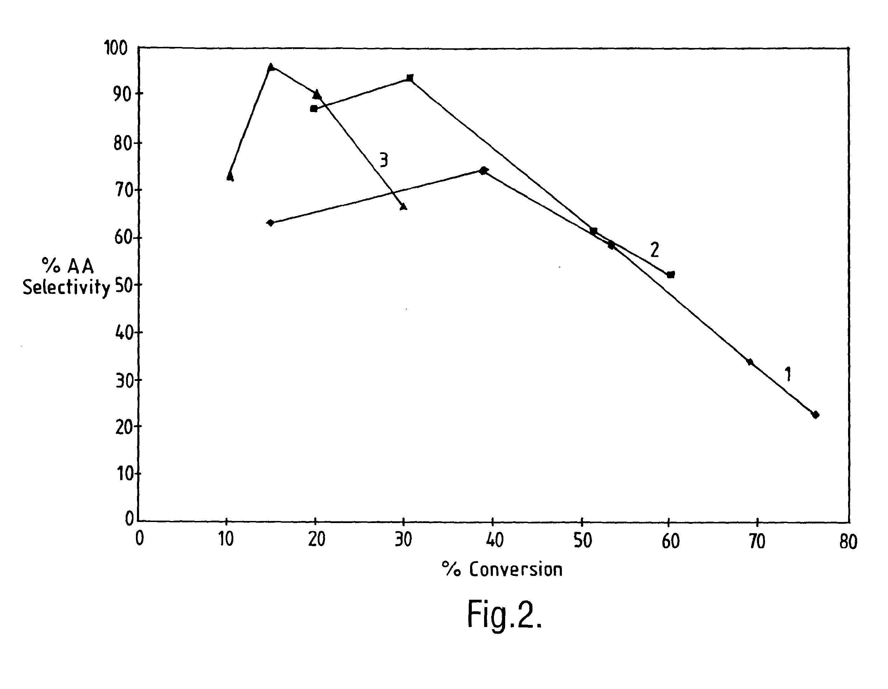

An aqueous solution containing ammonium heptamolybdate tetrahydrate, ammonium metavanadate and telluric acid was prepared by dissolving the corresponding salts in water at 70C°. The so-formed solution was cooled to 40°C and an aqueous solution of niobium oxalate was added thereto. After removing the water therefrom at 50°C, under 80 to 30 mmHg, the solid materials were further dried in a vacuum oven at room temperature overnight. The dried materials were placed in a one-inch diameter quartz tube and the quartz tube was plugged at each end with quartz wool. The quartz tube was then placed in a tube furnace in a vertical orientation and gas lines were then connected to the tube. A flow of 8% O₂ in N₂ was then introduced into the bottom of the tube at a flow rate of 100 ccm. The furnace was then heated at 10°C/min to 300°C and then the gas flow was switched to all nitrogen; heating was then continued at 2°C/min to 600°C and then held at that temperature for 2 hours. After cooling to room temperature, the final catalyst, which had a nominal composition of Mo₁V_{0.30}Te_{0.23}Nb_{0.08}, was pressed in a mold and then broken and sieved to 10 -20 mesh granules for reactor evaluation, i.e. the vapor phase catalytic oxidation of propane to acrylic acid. The reactor evaluation results are plotted as curve 1 (solid diamonds) in Fig. 2.

### Example 3

An aqueous solution containing ammonium heptamolybdate tetrahydrate, ammonium metavanadate and telluric acid was prepared by dissolving the corresponding salts in water at 70C°. The so-formed solution was cooled to 40°C and an aqueous solution of niobium oxalate was added thereto. After removing the water therefrom at 50°C, under 80 to 30 mmHg, the solid materials were further dried in a vacuum oven at room temperature overnight. The dried materials were placed in a one-inch diameter quartz tube and the quartz tube was plugged at each end with quartz wool. The quartz tube was then placed in a tube furnace in a vertical orientation and gas lines were then connected to the tube. A flow of 8% O₂ in N₂ was then introduced into the bottom of the tube at a flow rate of 100 ccm. The furnace was then heated at 10°C/min to 300°C and then the gas flow was switched to all nitrogen; heating was then continued at 2°C/min to 650°C and then held at that temperature for 2 hours. After cooling to room temperature, the final catalyst, which had a nominal composition of Mo₁V_{0.30}Te_{0.23}Nb_{0.08}, was pressed in a mold and then broken and sieved to 10 -20 mesh granules for reactor evaluation, i.e. the vapor phase catalytic oxidation of propane to acrylic acid. The reactor evaluation results are plotted as curve 2 (solid squares) in Fig. 2.

### Example 4

An aqueous solution containing ammonium heptamolybdate tetrahydrate, ammonium metavanadate and telluric acid was prepared by dissolving the corresponding salts in water at 70C°. The so-formed solution was cooled to 40°C and an aqueous solution of niobium oxalate was added thereto. After removing the water therefrom at 50°C, under 80 to 30 mmHg, the solid materials were further dried in a vacuum oven at room temperature overnight. The dried materials were placed in a one-inch diameter quartz tube and the quartz tube was plugged at each end with quartz wool. The quartz tube was then placed in a tube furnace in a vertical orientation and gas lines were then connected to the tube. A flow of 8% O₂ in N₂ was then introduced into the bottom of the tube at a flow rate of 100 ccm. The furnace was then heated at 10°C/min to 300°C and then the gas flow was switched to all nitrogen; heating was then continued at 2°C/min to 675°C and then held at that temperature for 2 hours. After cooling to room temperature, the final catalyst, which had a nominal composition of Mo₁V_{0.30}Te_{0.23}Nb_{0.08}, was pressed in a mold and then broken and sieved to 10 -20 mesh granules for reactor evaluation, i.e. the vapor phase catalytic oxidation of propane to acrylic acid. The reactor evaluation results are plotted as curve 3 (solid triangles) in Fig. 2.

## Claims

1. An improved process for preparing a catalyst including a compound having the formula
AₐV_{b}N_{c}X_{d}Oₑ
wherein, when a = 1, b = 0.1 to 1, c = 0.1 to 1, d = 0.01 to 1 and e is dependent on the oxidation state of the other elements, and
wherein A is at least one element selected from the group consisting of Mo and W, N is at least one element selected from the group consisting of Te, Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb, said process comprising:
admixing metal compounds, at least one of which is an oxygen containing compound, and at least one solvent to form a solution;
removing the solvent from the solution to obtain a catalyst precursor; and
calcining the catalyst precursor to form said catalyst;
wherein the improvement comprises:
(A) admixing said metal compounds, at least one of which is an oxygen containing compound, and said at least one solvent to form a solution,
(B) removing said at least one solvent from said solution to obtain a catalyst precursor, and
(C) calcining said catalyst precursor at a temperature of at least 650°C in a non-oxidizing atmosphere to form a catalyst including a compound having the formula
AₐV_{b}N_{c}X_{d}Oₑ
wherein, when a = 1, b = 0.1 to 1, c = 0.1 to 1, d = 0.01 to 1 and e is dependent on the oxidation state of the other elements, and
wherein A is at least one element selected from the group consisting of Mo
and W, N is at least one element selected from the group consisting of Te,
Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb.

2. The process according to claim 1, wherein said catalyst precursor is calcined at a temperature of from at least 650°C to 750°C.

3. The process according to claim 1, wherein said catalyst precursor is calcined at a temperature of from at least 650°C to 700°C.

4. The process according to claim 1, wherein N is at least one element selected from the group consisting of Te and Sb; and X is at least one element selected from the group consisting of Nb, Ta and Zr.

5. The process according to claim 1, wherein A is Mo, N is Te, and X is Nb.

6. The process according to claim 1, wherein said non-oxidizing atmosphere comprises an inert gas.

7. The process according to Claim 1, wherein said non-oxidizing atmosphere flows over said catalyst precursor.

8. An improved catalyst prepared according to the process of Claim 1.

9. An improved process for preparing a catalyst including a compound having the formula
AₐV_{b}N_{c}X_{d}Oₑ
wherein, when a = 1, b = 0.1 to 1, c = 0.1 to 1, d = 0.01 to 1 and e is dependent on the oxidation state of the other elements, and
wherein A is at least one element selected from the group consisting of Mo and W, N is at least one element selected from the group consisting of Te, Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb, said process comprising:
admixing metal compounds, at least one of which is an oxygen containing compound, and at least one solvent to form a solution;
removing the solvent from the solution to obtain a catalyst precursor; and
calcining the catalyst precursor to form said catalyst;
wherein the improvement comprises:
(A) admixing said metal compounds, at least one of which is an oxygen containing compound, and said at least one solvent to form a solution,
(B) removing said at least one solvent from said solution to obtain a catalyst precursor, and
(C) heating said catalyst precursor to a first temperature in an oxidizing atmosphere, then heating the so-treated catalyst precursor from said first temperature to a second temperature in a non-oxidizing atmosphere to form a catalyst including a compound having the formula
AₐV_{b}N_{c}X_{d}Oₑ
wherein, when a = 1, b = 0.1 to 1, c = 0.1 to 1, d = 0.01 to 1 and e is dependent on the oxidation state of the other elements, and
wherein A is at least one element selected from the group consisting of Mo
and W, N is at least one element selected from the group consisting of Te,
Se and Sb, and X is at least one element selected from the group consisting of Nb, Ta, Ti, Al, Zr, Cr, Mn, Fe, Ru, Co, Rh, Ni, Pd, Pt, Bi, B, In, Ce, As, Ge, Sn, Li, Na, K, Rb, Cs, Fr, Be, Mg, Ca, Sr, Ba, Ra, Hf, Ag, Pb, P, Pm, Eu, Gd, Dy, Ho, Er, Tm, Yb, Lu, Au, Re, Pr, Zn, Ga, Pd, Ir, Nd, Y, Sm and Tb.

10. The process according to Claim 9, wherein said catalyst precursor is heated to a first temperature of from 200°C to 400°C in a flowing oxidizing atmosphere; said catalyst precursor is held at said first temperature, in said flowing oxidizing atmosphere, for from 15 minutes to 8 hours; the so-treated catalyst precursor is then heated from said first temperature to a second temperature of at least 500°C in a flowing non-oxidizing atmosphere; and the so-treated catalyst precursor is held at the second temperature, in said flowing non-oxidizing atmosphere, for from 15 minutes to 8 hours to form said catalyst.

11. The process according to Claim 10, wherein said first temperature is from 275°C to 325°C; said catalyst precursor is held at said first temperature, in said flowing oxidizing atmosphere, for from 1 to 3 hours; said second temperature is from 650°C to 700°C; and the so-treated catalyst precursor is held at said second temperature, in said flowing non-oxidizing atmosphere, for from 1 to 3 hours.

12. The process according to Claim 9, wherein said oxidizing atmosphere comprises oxygen gas and said non-oxidizing atmosphere comprises an inert gas.

13. An improved catalyst prepared according to the process of Claim 9.

14. A process for preparing an unsaturated aldehyde or carboxylic acid comprising subjecting an alkane to catalytic oxidation in the presence of a catalyst prepared by the process of claim 1.

15. A process for preparing an unsaturated aldehyde or carboxylic acid comprising subjecting an alkane to catalytic oxidation in the presence of a catalyst prepared by the process of claim 9.

16. A process for preparing an unsaturated nitrile comprising subjecting an alkane to catalytic oxidation in the presence of ammonia and a catalyst prepared by the process of claim 1.

17. A process for preparing an unsaturated nitrile comprising subjecting am alkane to catalytic oxidation in the presence of ammonia and a catalyst prepared by the process of claim 9.
